(19) Europäisches Patentamt
European Patent Office
Office européen des brevets

(11) Veröffentlichungsnummer : **0 131 254**
**B1**

(12) # EUROPÄISCHE PATENTSCHRIFT

(45) Veröffentlichungstag der Patentschrift : **25.05.88**

(51) Int. Cl.⁴ : **C 07 D307/06**// C07C69/63, C07C33/42

(21) Anmeldenummer : **84107786.0**

(22) Anmeldetag : **04.07.84**

(54) **Verfahren zur Herstellung substituierter Tetrahydrofurane.**

(30) Priorität : **08.07.83 CH 3754/83**

(43) Veröffentlichungstag der Anmeldung : **16.01.85 Patentblatt 85/03**

(45) Bekanntmachung des Hinweises auf die Patenterteilung : **25.05.88 Patentblatt 88/21**

(84) Benannte Vertragsstaaten : **CH DE FR GB LI NL**

(56) Entgegenhaltungen :
**DE-A- 1 493 885**
**US-A- 3 122 587**
**TETRAHEDRON LETTERS, Band 21, Nr. 5, 1980, Seiten 441-444, Pergamon Press Ltd., GB; S.G. HEDGE et al.: "The reaction of hypochlorous acid with olefins. A convenient synthesis of allylic chlorides"**
**JOURNAL OF ORGANIC CHEMISTRY, Band 46, Nr. 16, 31. Juli 1981, Seiten 3349-3352, Columbus, Ohio, USA; J.J. FITT et al.: "Lithiation of N,N-dimethylmethallylamine"**
**Die Akte enthält technische Angaben, die nach dem Eingang der Anmeldung eingereicht wurden und die nicht in dieser Patentschrift enthalten sind.**

(73) Patentinhaber : **L. GIVAUDAN & CIE Société Anonyme**
**CH-1214 Vernier-Genève (CH)**

(72) Erfinder : **Naegeli, Peter, Dr.**
**Vordere Höhenstrasse 31**
**CH-5430 Wettingen (CH)**

(74) Vertreter : **Lederer, Franz, Dr. et al**
**Van der Werth, Lederer & Riederer Patentanwälte**
**Lucile-Grahn-Strasse 22**
**D-8000 München 80 (DE)**

**EP 0 131 254 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein neues Verfahren zur Herstellung von gewissen Tetrahydrofuranderivaten, nämlich von Verbindungen der allgemeinen Formel (I)

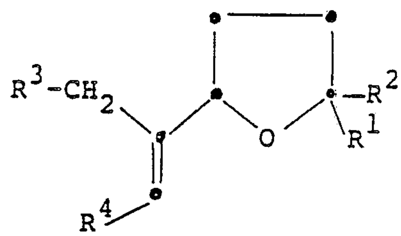

(I)

worin

$R^1$ Wasserstoff, $C_{1\text{-}6}$-Alkyl oder $C_{2\text{-}6}$-Alkenyl,

$R^2$ $C_{1\text{-}3}$-Alkyl und

$R^3$ und $R^4$, unabhängig voneinander Wasserstoff oder $C_{1\text{-}3}$-Alkyl bedeuten,

welche Verbindungen grösstenteils bekannt sind, und zwar insbesondere als Riechstoffe.

Das erfindungsgemässe Verfahren ist dadurch gekennzeichnet, dass man eine Verbindung der allgemeinen Formel (II)

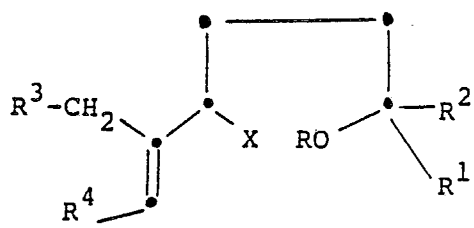

(II)

worin

$R^1$-$R^8$ die oben angegebenen Bedeutungen besitzen,

R Wasserstoff, Formyl, $C_2$-$C_7$ Alkanoyl oder Aroyl bedeutet und

X Chlor oder Brom bedeutet,

mit einer Base behandelt.

In der obigen Definition der Substituenten $R^1$-$R^4$ umfassen die Ausdrücke « Alkyl » und « Alkenyl » sowohl geradkettige als auch verzweigte Alkyl- bzw. Alkenylgruppen. $C_{2\text{-}6}$-Alkenyl ist vorzugsweise Vinyl. Unter « $C_2$-$C_7$ Alkanoyl » sind diejenigen Alkanoylgruppen zu verstehen, in denen der Alkylteil 1 bis 6 Kohlenstoffatome enthält. Der Ausdruck « Aroyl » umfasst insbesondere Benzoyl und dessen einfach substituierte Derivate, z. B. mit Methyl substituiertes Benzoyl.

Die Formeln (I) und (II) sollen alle möglichen isomeren Verbindungen dieser Formeln sowie deren Gemische umfassen.

Durch die Basenbehandlung wird die Verbindung der Formel (II) einem Ringschluss unterworfen und die Verbindung der Formel (I) gebildet. Zu diesem Zwecke eignen sich als Basen sowohl anorganische als auch organische Basen, wobei erstere bevorzugt sind. Zu den Basen gehören Alkalimetall- und Erdalkalimetallhydroxide, -carbonate und -bicarbonate, wie Natriumhydroxid, Kaliumhydroxid, Magnesiumhydroxid, Lithiumcarbonat, Natriumcarbonat, Kaliumcarbonat und Calciumcarbonat ; Alkalimetall- und Erdalkalimetallalkoxylate, wie Kalium-tert. butoxid ; und primäre, sekundäre und tertiäre Amine, wie Aethylamin, Butylamin, Diäthylamin, Diisopropylamin, Triäthylamin und Pyridin. Bevorzugt sind insbesondere Natriumhydroxid, Kaliumhydroxid, Magnesiumhydroxid, Lithiumcarbonat, Natriumcarbonat, Kaliumcarbonat, Calciumcarbonat und Kalium-tert.-butoxid.

Die Umsetzung wird in Gegenwart eines inerten organischen Lösungsmittels, durchgeführt. Als Verdünnungsmittel eignen sich insbesondere gesättigte aliphatische und alicyclische sowie aromatische Kohlenwasserstoffe, wie n-Pentan, n-Hexan, Cyclohexan, Benzol, Toluol und Xylole ; aliphatische und cyclische Aether, wie Diäthyläther, Glykol-mono- und diäther (z. B. Glykolmethyläther und -dimethyläther), Tetrahydrofuran und Dioxan ; Alkohole, wie Methanol, Aethanol und Isopropanol ; sowie aprotische Lösungsmittel mit hoher Dielektrizitätskonstante, wie Dimethylformamid, N-Methylpyrrolidon, N-substituierte Harnstoffe, Dimethylsulfoxid und Sulfolan. Bevorzugt sind Alkohole und aliphatische und cyclische Aether.

Zur Beschleunigung der Reaktion ist es oft vorteilhaft, sogenannte Phasentransferkatalysatoren zuzufügen. Beispiele solcher Katalysatoren sind quaternäre Ammoniumsalze, Phosphoniumsalze und

Polyäthylenglykoläther-Kronenverbindungen.

Die Behandlung der Verbindung der Formel (I) mit einer Base wird zweckmässigerweise bei Temperaturen zwischen — 20 °C und 150 °C, bevorzugt bei Temperaturen zwischen Raumtemperatur und 100 °C, durchgeführt.

Isolierung und Reinigung der so erhaltenen Verbindungen der Formel (I) können in an sich bekannter Weise erfolgen, z. B. durch Extrahieren und Destillation bzw. Chromatographie.

Sofern keine gezielte Synthese zur Isolierung reiner Isomerer durchgeführt wird, fällt normalerweise ein Produkt als Gemisch zweier oder mehrerer Isomerer an. Die Isomeren können nach an sich bekannten Methoden aufgetrennt werden. Gewünschtenfalls können z. B. die optischen Isomeren durch Einsatz entsprechender optisch aktiver Ausgangsmaterialien hergestellt werden.

Gegenüber bisherigen Verfahren zur Herstellung von allyl-substituierten Tetrahydrofuranen, z. B. aus Diolen durch Säurebehandlung oder durch Monomesylierung und Behandlung mit tertiären Aminen, ist das erfindungsgemässe Verfahren in basischem Milieu und bei milden Temperaturen schonender, das Endprodukt, ein allylischer Aether, wird weder durch Säure noch durch hohe Temperaturen belastet, welche Faktoren bekanntlich die Stabilität solcher allylischer Aether beeinflussen : Das Endprodukt wird dadurch in geruchlich optimalem Zustand erhalten (siehe diesbezüglich z. B. dragoco report 6/7-1980, Seiten 159-164).

Umsetzungen von Halogeniden mit Basen zu cyclischen Aethern sind aus

(1) Tetr. Lett., 21, (1980), Seiten 441-444

(2) J. Org. Chem., 46, (1981), Seiten 3349- 3352

(3) US-A-3122 587

bekannt geworden.

Im Falle von (2) und (3) sind jedoch primäre Halogenide involviert ; im Falle von (1) führt die Umsetzung des sekundären Halogenids mit der Base zur Elimination von Cl als HCl unter Ausbildung einer Doppelbindung, in Konjugation zur schon bestehenden Doppelbindung ; es resultiert ein Dienol. Dieses Dienol wird anschliessend in einer weiteren Stufe einem üblichen sauren Aetherringschluss unterworfen, wobei ein Pyran entsteht.

Vorzugsweise wird das erfindungsgemässe Verfahren benützt zur Herstellung von :

2-Isopropenyl-5-methyl-5-vinyl-tetrahydrofuran ausgehend von 6-Acetoxy-3-Chlor-2,6-dimethyl-octa-1,7-dien oder 3-Chlor-2,6-dimethyl-octa-1,7-dien-6-ol ;

2-Aethyl-5-isopropenyl-2-methyl-tetrahydrofuran ausgehend von 6-Acetoxy-3-chlor-2,6-dimethyl-1-octen ;

2-Methyl-5-(1-methyliden-propyl)-2-vinyl-tetrahydrofuran ausgehend von 3-Acetoxy-6-chlor-3-methyl-7-methyliden-1-nonen ;

2-Isopropenyl-5-methy-tetrahydrofuran ausgehend von 6-Acetoxy-3-Chlor-2-methyl-1-hepten ; und

2,2-Dimethyl-5-isopropenyl-tetrahydrofuran ausgehend von 6-Acetoxy-3-chlor-2,6-dimethyl-1-hepten.

Die als Ausgangsmaterialien verwendeten Allylhalogenide der Formel (II) sind bekannt oder können nach an sich bekannten Methoden, z. B. gemäss S.G. Hegde et al., Tetrahedron Letters 21, 441-444 (1980) oder PCT-Patentpublikation Nr. WO 82/00030, oder gemäss W. Sato et al., Chemistry Letters 1982, 141-142, hergestellt werden, nämlich durch Halogenierung und Umlagerung der Doppelbindung von substituierten Olefinen.

Die erste der angesprochenen Methoden zur Herstellung der Ausgangsmaterialien (II) besteht darin, dass man eine Verbindung der allgemeinen Formel (III)

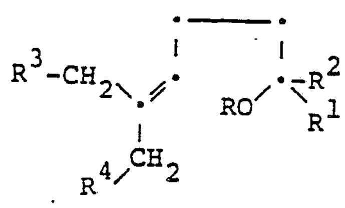

(III)

worin R und $R^1$-$R^4$ die oben angegebenen Bedeutungen besitzen,
mit unterchloriger bzw. unterbromiger Säure in Gegenwart von Methylenchlorid als Lösungsmittel umsetzt. Das Reagenz kann in diesem Verfahren in situ erzeugt werden, und zwar geeigneterweise aus Javell-Lauge [wässriges KOCl oder NaCl] (gegebenenfalls in Gegenwart von Lithiumbromid) oder aus Calciumhypochlorit durch Zugabe einer Säure, insbesondere Essigsäure oder wässriger prim. Alkalimetallphosphatlösung. Die Umsetzung erfolgt im allgemeinen bei Temperaturen zwischen — 30 °C und 35 °C, vorzugsweise zwischen — 10 °C und 25 °C, also unter milden Reaktionsbedingungen.

Die zweite Methode zur Herstellung von Verbindungen der allgemeinen Formel (III) besteht darin, dass man eine Verbindung der allgemeinen Formel (III) mit tert. Butylhypochlorit, vorzugsweise in Gegenwart von Kieselgel, umsetzt. Die Umsetzung wird zweckmässigerweise in Gegenwart eines organischen Lösungsmittels, insbesondere eines aliphatischen Kohlenwasserstoffes, wie n-Pentan oder n-Hexan ; eines chlorierten aliphatischen Kohlenwasserstoffes, wie Methylenchlorid oder Chloroform ;

oder eines aliphatischen oder cyclischen Aethers, wie Diäthyläther, Tetrahydrofuran oder Dioxan, durchgeführt. Es wird im allgemeinen bei Temperaturen zwischen — 20 °C und Raumtemperatur, vorzugsweise zwischen — 5 °C und 10 °C, gearbeitet. Diese Methode beschränkt sich auf die Herstellung denjenigen Verbindungen der Formel (II), in denen X Chlor bedeutet.

Eine weitere Methode zur Herstellung von Verbindungen der allgemeinen Formel (II) mit X = Chlor besteht darin, dass man eine Verbindung der Formel (III) mit Sulfurylchlorid in Gegenwart eines tertiären Amins als Base umsetzt. Beispiele solcher Basen sind Triäthylamin, Dimethylanilin und Pyridin. Zudem wird die Umsetzung zweckmässigerweise in Gegenwart eines organischen Lösungsmittels, insbesondere eines gesättigten aliphatischen oder alicyclischen Kohlenwasserstoffes, wie n-Pentan, n-Hexan und Cyclohexan ; oder eines chlorierten Kohlenwasserstoffes, wie Methylenchlorid, Chloroform, Tetrachlorkohlenstoff und Chlorbenzole, durchgeführt. Die Reaktionstemperaturen liegen im allgemeinen zwischen — 40 °C und 60 °C, vorzugsweise zwischen — 20 °C und 20 °C.

Die Isolierung und die Reinigung (falls notwendig) der so erhaltenen Verbindungen der Formel (II) können in an sich bekannter Weise erfolgen, z. B. Destillation oder Chromatographie.

Die nachfolgenden Beispiele dienen zur näheren Erläuterung der Erfindung.

Beispiel 1

Herstellung von 2-Isopropenyl-5-methyl-5-vinyl-tetrahydrofuran

A. Zu einer Lösung von 227 g (0,98 Mol) 3-Acetoxy-6-chlor-3,7-dimethyl-octa-1,7-dien in 500 ml Methanol werden 270 ml 30 %-ige Natronlauge (ca. 2 Mol NaOH) und 1,5 g Tricaprylylmethylammoniumchlorid (« Aliquat 336 ») bei 10 °C zugegeben. Man rührt das Reaktionsgemisch kräftig während 3-4 Stunden bei Raumtemperatur und anschliessend noch während 3 Stunden bei Rückflusstemperatur.

Zur Aufarbeitung verdünnt man das Gemisch mit 0,7-1 l Wasser, extrahiert das verdünnte Gemisch mit 0,25-0,5 Teilen n-Pentan, wäscht die organische Phase mit Wasser neutral, trocknet sie über wasserfreiem Magnesiumsulfat und dampft sie sorgfältig bei tiefstmöglicher Temperatur ein. Es bleiben 96,8 g einer leicht gelblichen Flüssigkeit zurück, die man in Gegenwart von 0,5-1,0 g trockener Pottasche in einer Vigreuxkolonne (17 cm lang) einer fraktionierten Destillation unterwirft. Die bei 74-84 °C/150 mm Hg destillierten Fraktionen stellen reines Produkt dar und wiegen insgesamt 72,5 g. Das so erhaltene 2-Isopropenyl-5-methyl-5-vinyl-tetrahydrofuran besteht aus einem Gemisch des cis- und trans-Isomeren.

IR (Film) : 3 090, 1 648, 1 450, 1 400, 1 370, 1 300, 1 280, 1 250/1 240, 1 130, 1 100, 1 050, 1 035, 1 000, 925, 900, 700 $cm^{-1}$.

$^1$H—NMR ($CDCl_3$/$(CH_3)_4Si$ ; 60 MHz) :

$\delta$ = 5,98 ppm (dd $J_1$ = 17 Hz, $J_2$ = 10 Hz ; cis-Verbindung), $\delta$ = 5,90 ppm (dd $J_1$ = 17 Hz, $J_2$ = 10 Hz ; trans-Verbindung), $\delta$ = 5,20 ppm (breites d J = 17 Hz, $\delta$ = 4,98 ppm (breites d J = 10 Hz), $\delta$ = 5,02 ppm (breites s), $\delta$ = 4,80 ppm (breites s), $\delta$ = 4,40 ppm (breites t), $\delta$ = 1,72 ppm (s), $\delta$ = 1,33 ppm und 1,31 ppm (2 × s ; cis- und trans-Verbindung).

Das als Ausgangsmaterial benötigte 3-Acetoxy-6-chlor-3,7-dimethyl-octa-1,7-dien kann gemäss der nachfolgenden Methode a) oder b) hergestellt werden :

a) Eine Lösung von 4,8 ml (60 mMol) Sulfurylchlorid in 20 ml trockenem n-Hexan wird bei — 20 °C unter Rühren zu 9,8 g (50 mMol) Linalylacetat (3-Acetoxy-3,7-dimethyl-octa-1,6-dien) in 100 ml trockenem n-Hexan und 5,6 ml (70 mMol) trockenem Pyridin zugetropft, wobei ein feiner weissfarbener Niederschlag von Pyridinhydrochlorid gebildet wird. Das Reaktionsgemisch wird weitere 30 Minuten bei — 20 °C gerührt und anschliessend mit 200 ml n-Hexan verdünnt. Dann wird das Gemisch mit ca. 500 ml Wasser neutral gewaschen, und die wässrigen Phasen werden mit 100 ml n-Hexan nachextrahiert. Die vereinigten organischen Phasen werden über wasserfreiem Magnesiumsulfat getrocknet und unter vermindertem Druck eingeengt. Man erhält 10,0 g (86,5 % der theoretischen Ausbeute) 3-Acetoxy-6-chlor-3,7-dimethyl-octa-1,7-dien als klare Flüssigkeit, deren spektrale Daten zur Identifizierung dienen.

b) 196 g (1 Mol) Linalylacetat (95 % rein) werden in 1 l Methylenchlorid gelöst, worauf 600 ml Wasser und 121 g (0,55 Mol) Calciumhypochrit (65-70 % rein) zugegeben werden. In das Gemisch werden unter gutem Rühren 96,8 ml (1,7 Mol) Eisessig langsam zugetropft, wobei die Temperatur des Gemisches mit Hilfe eines Eisbades auf 10-15 °C gehalten wird. Nach Beendigung der Zugabe (ca. 30 Minuten) lässt man das Reaktionsgemisch bei Raumtemperatur weitere 45 Minuten rühren. Der pH-Wert der Lösung beträgt ca. 4,5.

Zur Aufarbeitung wäscht man das Gemisch mit Wasser neutral und extrahiert die wässrigen Phasen zweimal mit jeweils 500 ml Methylenchlorid. Dann werden die vereinigten organischen Phasen über wenig wasserfreiem Magnesiumsulfat getrocknet und nach Filtration eingedampft. Die Ausbeute an Rohprodukt, einer klaren, fast farblosen Flüssigkeit, beträgt 227,5 g (98,6 % der theoretischen Ausbeute). Es wird durch sein IR- und NMR-Spektrum als 3-Acetoxy-6-chlor-3,7-dimethyl-octa-1,7-dien identifiziert.

IR (Film) : 3 090, 1 735, 1 642, 1 375, 1 250, 1 178, 1 110, 1 025, 920/910, 745 $cm^{-1}$.

$^1$H—NMR ($CDCl_3$ und $(CH_3)_4Si$ ; 400 MHz) :

$\delta$ = 5,95 ppm und 5,92 ppm (2 × dd $J_1$ = 17 Hz, $J_2$ = 11 Hz), $\delta$ = 5,165 ppm und 5,155 ppm (2 ×

feingespaltenes d J = 17 Hz), δ = 5,14 ppm (feingespaltenes d J = 11 Hz), δ = 5,01 ppm und 4,90 ppm (2 × breites s), δ = 4,34 ppm (Pseudo-t J = 7 Hz), δ = 2,01 ppm (s), δ = 1,79 ppm (s), δ = 1,55 ppm und 1,54 ppm (2 × s ; cis- und trans-Verbindung).

B. Eine Lösung von 27 g (0,14 Mol) 3-Chlor-2,6-dimethyl-octa-1,7-dien-6-ol in 100 ml n-Pentan wird so unter gutem Rühren zu 50 ml 30 %-iger Natronlauge, die 2,3 g (10 mMol) Triäthylbenzylammoniumchlorid enthält und mit 50 ml n-Pentan überschichtet ist, zugetropft, dass keine übermässige Erwärmung auftritt. Zum Nachspülen werden weitere 50 ml n-Pentan zugegeben. Nach Beendigung der Zugabe lässt man das Reaktionsgemisch einige Stunden bei Raumtemperatur weiterreagieren. Dann trennt man die wässrige Phase ab und extrahiert sie mit 100 ml n-Pentan. Die vereinigten organischen Phasen werden mit genügend Wasser neutral gewaschen, und nach Trocken über wasserfreiem Magnesiumsulfat und Filtration wird das Lösungsmittel von der organischen Phase sorgfältig abgedampft. Das verbleibende Rohprodukt wird über eine kleinere Füllkörper- oder in einer Widmerkolonne fraktioniert destilliert. Die bei 36 °C/0,12 mm Hg destillierte Fraktion besteht aus 11-12 g cis/trans-Gemisch von 2-Isopropenyl-5-methyl-5-vinyl-tetrahydrofuran. Die spektralen Daten dieses Produktes entsprechen den oben angegebenen.

Das als Ausgangsmaterial benötigte 3-Chlor-2,6-dimethyl-octa-1,7-dien-6-ol kann wie folgt hergestellt werden :

12,32 g (80 mMol) Linalool (3,7-Dimethyl-octa-1,6-dien-3-ol) werden in einem Scheidetrichter (2 l) in 400 ml Methylenchlorid gelöst, die Lösung wird mit einer Lösung von 8,5 g (etwas mehr als 80 mMol) 70 %-ige Calciumhypochlorit in 80 ml Wasser überschichtet. Zum Inhalt des Scheidetrichters gibt man Eis, dann schüttelt man das Reaktionsgemisch kräftig durch. Dazu fügt man kleine Portionen Trockeneispulver und hält so die Temperatur unter 20 °C. Man vermeidet eine Emulsionsbildung bei einem pH-Wert von 6-7 der wässrigen Phase, indem man kleine Mengen 2n Essigsäure zugibt. Die organische Phase wird mit genügend Wasser neutral gewaschen, über wasserfreiem Magnesiumsulfat getrocknet und nach Filtration unter vermindertem Druck eingedampft. Es resultiert ein farbloses Oel (14,6 g). Das Produkt wird ohne Reinigung verwendet.

IR (Film) : 3 400, 3 080, 1 645, 1 450, 1 415, 1 375, 1 120, 1 000, 925/910 $cm^{-1}$.

$^1H$—NMR ($CDCl_3/(CH_3)_4Si$ ; 60 MHz) :

Zwischen δ = 6,2 ppm und δ = 4,85 ppm : Merkmale für $CH_2$ = CH- und $CH_2$ = C- der beiden Diastereomeren, δ = 4,39 ppm (t J = 6 Hz, $CH_2$ = C—C$\underline{H}$—Cl), δ = 1,8 ppm (s, $CH_2$ = C (C$\underline{H}_3$)-), δ = 1,3 ppm (s, — C (C$\underline{H}_3$) (OH)).

## Beispiel 2

Herstellung von 2-Aethyl-5-isopropenyl-2-methyl-tetrahydrofuran

Zu einer Lösung von 6,5 g (0,03 Mol) 6-Acetoxy-3-chlor-2,6-dimethyl-1-octen in 50 ml Methanol werden 0,1 g Tricaprylylmethylammoniumchlorid (« Aliquat 336 ») sowie, tropfenweise bei 0-10 °C, 30 ml 30 %-ige Natronlauge (0,225 Mol NaOH) zugegeben. Nach beendeter Zugabe lässt man das milchig weisse Reaktionsgemisch während ca. 16 Stunden bei Raumtemperatur weiterreagieren. Falls im vorliegenden Fall kein Aliquat 336 benützt wird, muss das Reaktionsgemisch länger, beispielsweise 24 bis 48 Stunden gerührt werden.

Zur Aufarbeitung extrahiert man das Gemisch zweimal mit jeweils 25 ml n-Pentan, wäscht die vereinigten organischen Phasen mit genügend Wasser neutral, trocknet sie über wasserfreiem Magnesiumsulfat und dampft das Lösungsmittel sorgfältig ab. Es bleiben 3,6 g Rohprodukt zurück, das unter vermindertem Druck (11 mm Hg) destilliert wird. Auf diese Weise erhält man 2,8 g 2-Aethyl-5-isopropenyl-2-methyl-tetrahydrofuran als cis-/trans-Isomerengemisch, dessen Geruch camphrig, süss, an Kunsthonig erinnernd und fruchtig ist.

IR (Film) : 3 080, 1 650, 1 460, 1 375, 1 145, 1 110, 1 065, 900, 870 $cm^{-1}$.

Massenspektrum : $M^+/e$ = 154 (4), 139 (5), 125 (13), 121 (1), 111 (1), 107 (5), 95 (4), 82 (6), 67 (21), 57 (18), 55 (18), 43 (100), 29 (23).

$^1H$—NMR ($CDCl_3/(CH_3)_4Si$ ; 400 MHz) :

δ = 5,01 ppm und 4,78 ppm (2 × schmales m, C$\underline{H}_2$ = der cis- und trans-Verbindung), δ = 4,38 ppm und 4,25 ppm (2 × breites Pseudo-t J = 7 Hz, $CH_2$ = C—C$\underline{H}$-O), δ = 1,72 ppm (breites s, C$\underline{H}_3$—C—CH—O), δ = 1,21 ppm (2 fast zusammenfallende scharfe s, O—C—C$\underline{H}_3$ der cis- und trans-Verbindung), δ = 0,93 ppm und 0,91 ppm (2 × t J = 7 Hz, —$CH_2C\underline{H}_3$ der cis- und trans-Verbindung).

Das als Ausgangsmaterial benützte 6-Acetoxy-3-chlor-2,6-dimethyl-1-octen kann wie folgt hergestellt werden :

Zu einem Gemisch von 6,6 g (33,2 mMol) Dihydrolinalylacetat (6-Acetoxy-2,6-dimethyl-2-octen) in 100 ml Methylenchorid und 4,02 g (18,3 mMol) Calciumhypochlorit (ca. 65 % rein) in 50 ml Wasser werden bei einer Innentemperatur von 10 °C unter Rühren 3,2 ml (56 mMol) Eisessig innert 10 Minuten zugetropft. Darauf wird das Reaktionsgemisch langsam auf Raumtemperatur gebracht und noch ca. 16 Stunden bei dieser Temperatur weitergerührt. Das Gemisch wird mit Wasser neutral gewaschen und die wässrige Phase noch mit 50 ml Methylenchlorid extrahiert. Man trocknet die vereinigten organischen

Phasen über wasserfreiem Magnesiumsulfat und entfernt das Lösungsmittel nach Filtration unter vermindertem Druck und bei leicht erhöhter Temperatur. Es resultieren 6,9 g Rohprodukt, das ohne Reinigung verwendet werden kann.

IR (Film) : 3 080, 1 730, 1 645, 1 460, 1 370, 1 250, 1 185, 1 165, 1 130, 1 020, 950-940, 910, 850, 790, 740 $cm^{-1}$.

$^1$H—NMR ($CDCl_3/(CH_3)_4Si$ ; 60 MHz) :

δ = 5,05 ppm und 4,95 ppm (2 × breites s, $C\underline{H}_2$ =), δ = 4,5-4,2 ppm (2 nebeneinander liegende triplettartige m, —C$\underline{H}$—Cl), δ = 2,02 ppm (scharfes s, $C\underline{H}_3CO$), δ = 1,42 ppm (s, $CH_2 = C(C\underline{H}_3)$-), δ = 0,87 ppm (t J = 7 Hz, —$CH_2C\underline{H}_3$).

## Beispiel 3

Herstellung von 2-Methyl-5-(1-methyliden-propyl)-2-vinyl-tetrahydrofuran

15 g (61,3 mMol) 3-Acetoxy-6-chlor-3-methyl-7-methyliden-1-nonen und 0,5 g Tricaprylylmethylammoniumchlorid (« Aliquat 336 ») werden in 50 ml Methanol gelöst. Zur Lösung werden bei 10 °C unter gutem Rühren 27 ml 30 %-ige Natronlauge (ca. 0,2 Mol NaOH) sorgfältig zugetropft, wobei die exotherme Reaktion in Gang gebracht wird. Das Reaktionsgemisch wird weitere 16 Stunden bei Raumtemperatur gerührt.

Zur Aufarbeitung wird das Gemisch zweimal mit jeweils 50 ml n-Hexan extrahiert und die organische Phase mit genügend Wasser neutral gewaschen. Dann trocknet man die vereinigten organischen Phasen über wasserfreiem Magnesiumsulfat und dampft sie nach Filtration ein. Das resultierende, gelbe, flüssige Rohprodukt (8,8 g) wird bei 20 mm Hg auf 100 °C kurzwegdestilliert, wobei 1 g fester Rückstand zurückbleibt. Durch seine spektralen Daten wird das Destillat als 2-Methyl-5-(1-methyliden-propyl)-2-vinyl-tetrahydrofuran identifiziert. Das Produkt weist einen camphrigen, grünen, metallischen, fettigen und rettichartigen Geruch auf.

IR (Film) : 3 090, 1 650, 1 460, 1 415/1 405, 1 370, 1 130, 1 100, 1 080, 1 040/1 030, 995, 920, 900, 795 $cm^{-1}$.

Massenspektrum : $M^+/e$ = 166 (2), 151 (6), 137 (13), 133 (2), 123 (3), 121 (3), 119 (3), 110 (10), 105 (2), 96 (11), 93 (10), 82 (25), 81 (32), 68 (65), 67 (78), 55 (100), 43 (66), 27 (65).

$^1$H—NMR ($CDCl_3/(CH_3)_4Si$ ; 400 MHz) :

δ = 5,98 ppm und 5,89 ppm (2 × dd $J_1$ = 17 Hz, $J_2$ = 10-11 Hz, -C$\underline{H}$ = $CH_2$ der cis- und trans-Verbindung) δ = 5,23 ppm und 5,22 ppm (2 × dd $J_1$ = 17 Hz, $J_2$ = 1 Hz, trans-ständiges -CH = $C\underline{H}_2$ der cis- und trans-Verbindung), δ = 5,12 ppm und 5,09 ppm (2 × schmales m, >= $C\underline{H}_2$ des einen Isomeren), δ = 5,01 ppm und 4,99 ppm (2 × dd $J_1$ = 10 Hz, $J_2$ = 1 Hz, cis-ständiges -CH = $C\underline{H}_2$ der cis- und trans-Verbindung), δ = 4,81 ppm (schmales m, >= $C\underline{H}_2$ des anderen Isomeren), δ = 4,47 ppm und 4,43 ppm (2 × breites t J = 7 Hz, O—C$\underline{H}$—CH = $CH_2$ der cis- und trans-Verbindung), δ = 1,365 ppm und 1,335 ppm (2 × scharfes s, O—C($C\underline{H}_3$)— der cis- und trans-Verbindung), δ = 1,08 ppm (2 fast kongruente scharfe t J = 7 Hz, —$CH_2C\underline{H}_3$ der cis- und trans-Verbindung).

Das als Ausgangsmaterial benützte 3-Acetoxy-6-chlor-3-methyl-7-methyliden-1-nonen kann wie folgt hergestellt werden :

21,03 g (0,1 Mol) Aethyllinalylacetat (3-Acetoxy-3,7-dimethyl-nona-1,6-dien) werden in 100 ml Methylenchlorid gelöst. Ueber dieser Lösung wird dann eine Lösung von 12,1 g (0,055 Mol) Calciumhypochlorit (ca. 65 % rein) in 60 ml Wasser geschichtet, und unter gutem Rühren wird während 40 Minuten bei 10-15 °C 9,7 ml (0,17 Mol) Eisessig zugetropft. Man rührt das weisslich-trübe Reaktionsgemisch noch ca. 1 1/2 Stunden bei dieser Temperatur und anschliessend ca. 16 Stunden bei Raumtemperatur. Danach wird das Gemisch mit genügend Wasser neutral gewaschen und die organische Phase über wasserfreiem Magnesiumsulfat getrocknet und nach Filtration zu einer gelblichen Flüssigkeit eingedampft. Das 3-Acetoxy-6-chlor-3-methyl-7-methyliden-1-nonen (20,3 g) wird bei 85 °C/0,1 mm Hg destilliert.

IR (Film) : 3 080, 1 740, 1 645, 1 640, 1 460-1 440, 1 415, 1 370, 1 250, 1 175, 1 105, 1 020, 995, 930/920, 865, 800 $cm^{-1}$.

$^1$H—NMR ($CDCl_3/(CH_3)_4Si$ ; 60 MHz) :

δ = 5,98 ppm (2 sich fast deckende dd $J_1$ = 18 Hz, $J_2$ = 10 Hz, —C$\underline{H}$ = $CH_2$ der cis- und trans-Verbindung), δ = 5,14 ppm (Zentrum von je 2 fein aufgespaltenen d $J_1$ = 18 Hz, $J_2$ = 10 Hz, —CH = $C\underline{H}_2$, δ = 4,36 ppm (m, $CH_2$ = C—C$\underline{H}$—Cl), δ = 2,02 ppm (s, $C\underline{H}_3CO$—), δ = 1,55 ppm (s, —C ($C\underline{H}_3$) ($OCOCH_3$)), δ = 1,10 ppm (t J = 7 Hz, — $CH_2C\underline{H}_3$).

## Beispiel 4

Herstellung von 2-Isopropenyl-5-methyl-tetrahydrofuran

Zu einer Lösung von 5,5 g (27 mMol) 6-Acetoxy-3-chlor-2-methyl-1-hepten in 30 ml Methanol werden 0,1 g Tricaprylylmethylammoniumchlorid (« Aliquat 336 ») sowie langsam 15 ml 30 %-ige Natronlauge (ca. 0,1 Mol NaOH) bei einer Innentemperatur von 10 °C zugegeben, wobei wegen starker exothermer Reaktion gekühlt werden muss. Man rührt das Reaktionsgemisch bei Raumtemperatur während

16 Stunden.

Zur Aufarbeitung extrahiert man das trübweisse Gemisch zweimal mit jeweils 50 ml n-Pentan, wäscht die vereinigten organischen Phasen mit genügend Wasser neutral, trocknet sie über wasserfreiem Magnesiumsulfat und dampft sie nach Filtration sorgfältig ein. Es resultieren 2,35 g flüssiges Rohprodukt, das man bei ca. 60 °C/11 mm Hg kurzwegdestilliert. Die so erhaltene klare farblose Flüssigkeit (1,1 g) wird durch spektrale Daten als 2-Isopropenyl-5-methyl-tetrahydrofuran identifiziert. Dieses weist einen metallischen, bitteren, chemischen, ascaridolartigen, an Senföl und an Meerrettich erinnernden Geruch auf.

IR (Film) : 3 080, 1 650, 1 450, 1 380, 1 325, 1 190, 1 085, 1 030, 1 015, 900, 875 $cm^{-1}$.

Massenspektrum : $M^+/e$ = 126 (13), 111 (44), 98 (3), 93 (4), 85 (11), 77 (3), 70 (22), 67 (45), 55 (75), 41 (100), 27 (57).

$^1H$—NMR ($CDCl_3/(CH_3)_4Si$ ; 400 MHz) :

$\delta$ = 5,0 ppm und 4,97 ppm und auch $\delta$ = 4,79 ppm und 4,77 ppm (je 2 schmale m, = $C\underline{H}_2$ der cis- und trans-Verbindung), $\delta$ = 4,415 ppm und 4,27 ppm (2 × Pseudo-t, J = 8 Hz, $CH_2$ = C—C$\underline{H}$—O), $\delta$ = 4,16 ppm und 4,035 ppm (je t × d $J_1$ = 6 Hz, $J_2$ = 8 Hz, —C$\underline{H}$—O), $\delta$ = 1,72 ppm und 1,705 ppm (2 × s, $CH_2$ = C ($C\underline{H}_3$)- der cis- und trans-Verbindung), $\delta$ = 1,275 ppm und 1,25 ppm (2 × d J = 6 Hz, O—CH—$C\underline{H}_3$).

Das als Ausgangsmaterial benötigte 6-Acetoxy-3-chlor-2-methyl-1-hepten kann wie folgt hergestellt werden :

Ueber einer Lösung von 5,5 g (32 mMol) 6-Acetoxy-2-methyl-hept-2-en in 50 ml Methylenchlorid wird eine Lösung von 3,87 g (17,6 mMol) Calciumhypochlorit (ca. 65 % rein) in 20 ml Wasser geschichtet. Man tropft dann unter intensivem Rühren bei einer Innentemperatur von 5-10 °C 3 ml (54,4 mMol) Eisessig langsam zu. Das Kühlbad wird entfernt und das Reaktionsgemisch 2 Stunden weitergerührt. Nach Beendigung der Reaktion wird das Gemisch mit genügend Wasser neutral gewaschen und die organische Phase über wasserfreiem Magnesiumsulfat getrocknet und nach Filtration eingedampft. Man erhält 5,8 g 6-Acetoxy-3-chlor-2-methyl-1-hepten als klare Flüssigkeit.

IR (Film) : 1 738, 1 450, 1 375, 1 245, 1 135, 1 085, 1 050/1 020, 960, 910, 850, 742 $cm^{-1}$.

$^1H$—NMR ($CDCl_3/(CH_3)_4Si$ ; 60 MHz) :

$\delta$ = 5,05 ppm und 4,95 ppm (2 × schmales m, = $C\underline{H}_2$), $\delta$ = 4,65-4,2 ppm (m, $CH_2$ = C—C$\underline{H}$—Cl), $\delta$ = 2,04 ppm (s, $C\underline{H}_3CO$—), $\delta$ = 1,78 ppm (fein gespaltenes s, $CH_2$ = C($C\underline{H}_3$)—), $\delta$ = 1,23 ppm (d J = 7 Hz, —CH($C\underline{H}_3$)—).

## Beispiel 5

Herstellung von 2,2-Dimethyl-5-isopropenyl-tetrahydrofuran

12 g (55 mMol) 6-Acetoxy-3-chlor-2,6-dimethyl-1-hepten werden in 50 ml Methanol gelöst. Man gibt zur Lösung 0,1 g Tricaprylylmethylammoniumchlorid (« Aliquat 336 ») sowie unter Kühlen und unter gutem Rühren sorgfältig 27 ml 30 %-ige Natronlauge (ca. 0,2 Mol NaOH). Nach Beendigung der Zugabe lässt man das weisstrübe Reaktionsgemisch 16 Stunden bei Raumtemperatur weiterrühren.

Zur Aufarbeitung wird das Gemisch mit 50 ml n-Pentan extrahiert und die organische Phase mit genügend Wasser neutral gewaschen und über wasserfreiem Magnesiumsulfat getrocknet. Nach Filtration der organischen Phase wird das Lösungsmittel abgedampft. Die resultierende Flüssigkeit (4,6 g) wird dann bei 20 mm Hg und Temperaturen bis 150 °C destilliert. Ausbeute : 2 g 2,2-Dimethyl-5-isopropenyl-tetrahydrofuran ; Geruch : ätherisch, frisch, camphirg, naphthalinig.

IR (Film) : 3 080, 1 650, 1 455, 1 380, 1 368, 1 310, 1 290, 1 250, 1 230, 1 150, 1 070/1 050/1 030, 985, 950, 900, 865, 765 $cm^{-1}$.

Massenspektrum : $M^+/e$ = 140 (8), 125 (18), 107 (3), 97 (2), 91 (1), 81 (10), 70 (20), 67 (30), 55 (39), 43 (100), 27 (27).

$^1H$—NMR ($CDCl_3/(CH_3)_4Si$ ; 400 MHz) :

$\delta$ = 5,05 ppm und 4,78 ppm (2 × schmales m, = $C\underline{H}_2$), $\delta$ = 4,38 ppm (Pseudo-t J = 7 Hz, $CH_2$ = C—C$\underline{H}$—O), $\delta$ = 1,71 ppm (s, $CH_2$ = C($C\underline{H}_3$)—), $\delta$ = 1,27 ppm und 1,28 ppm (2 × s, C($C\underline{H}_3)_2$).

Das als Ausgangsmaterial benötigte 6-Acetoxy-3-chlor-2,6-dimethyl-1-hepten kann wie folgt hergestellt werden :

Eine Lösung von 12,9 g (0,07 Mol) 6-Acetoxy-2,6-dimethyl-2-hepten in 100 ml Methylenchlorid wird mit einer Lösung von 8,47 g (38,5 mMol) Calciumhypochlorit (ca. 65 % rein) in 50 ml Wasser überschichtet. Dann tropft man während 60 Minuten 6,72 ml (0,119 Mol) Eisessig unter gutem Rühren und bei einer Innentemperatur von 5-10 °C zu. Nach Beendigung der Zugabe lässt man das Reaktionsgemisch auf Raumtemperatur kommen und rührt es weitere 2 Stunden. Anschliessend wird das Gemisch mit genügend Wasser neutral gewaschen und die organische Phase über wasserfreiem Magnesiumsulfat getrocknet und nach Filtration unter vermindertem Druck eingedampft. Es resultieren 12,8 g rohes 6-Acetoxy-3-chlor-2,6-dimethyl-1-hepten.

IR (Film) : 3 080, 1 735, 1 645, 1 450, 1 385/1 370, 1 255, 1 200, 1 170, 1 130, 1 020, 950, 910, 745 $cm^{-1}$.

$^1H$—NMR ($CDCl_3/(CH_3)_4Si$ ; 60 MHz) :

$\delta$ = 5,03 ppm und 4,92 ppm (2 × breites s, = $C\underline{H}_2$), $\delta$ = 4,5-4,2 ppm (m, $CH_2$ = C—C$\underline{H}$-Cl), $\delta$ = 1,98 ppm (s, $C\underline{H}_3CO$—), $\delta$ = 1,8 ppm (s, $CH_2$ = C($C\underline{H}_3$)—, $\delta$ = 1,45 ppm (s, C($C\underline{H}_3)_2$).

**Patentansprüche**

1. Verfahren zur Herstellung von Verbindungen der allgemeinen Formel (I)

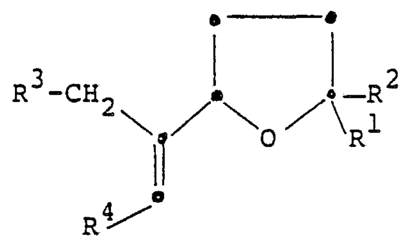

(I)

worin

$R^1$ Wasserstoff, $C_{1-6}$-Alkyl oder $C_{2-6}$-Alkenyl,

$R^2$ $C_{1-3}$-Alkyl und

$R^3$ und $R^4$ unabhängig voneinander Wasserstoff oder $C_{1-3}$-Alkyl bedeuten,

dadurch gekennzeichnet, dass man eine Verbindung der allgemeinen Formel (II)

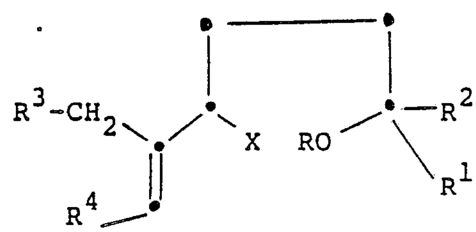

(II)

worin

$R^1$-$R^4$ die oben angegebenen Bedeutungen besitzen,

R Wasserstoff, Formyl, $C_{2-7}$-Alkanoyl oder Aroyl bedeutet und

X Chlor oder Brom bedeutet,

in Gegenwart eines inerten organischen Lösungsmittels mit einer Base behandelt.

2. Verfahren nach Anspruch 1, dadurch gekennzeichnet, dass dass man eine anorganische Base verwendet.

3. Verfahren nach Anspruch 1, dadurch gekennzeichnet, dass man eine organische Base verwendet.

4. Verfahren nach Anspruch 1, 2 oder 3, dadurch gekennzeichnet, dass die Umsetzung in einem Alkohol, einem aliphatischen Aether, einem cyclischen Aether, einem gesättigten aliphatischen oder alicyclischen Kohlenwasserstoff, oder einem aromatischen Kohlenwasserstoff durchgeführt wird.

5. Verfahren nach einem der Ansprüche 1 bis 4, dadurch gekennzeichnet, dass die Umsetzung in Gegenwart eines Phasentransferkatalysators durchgeführt wird.

6. Verfahren nach einem der Ansprüche 1 bis 5, dadurch gekennzeichnet, dass die Umsetzung bei Temperaturen zwischen der Raumtemperatur und 100 °C durchgeführt wird.

7. Verfahren nach einem der Ansprüche 1 bis 6, dadurch gekennzeichnet, dass man 6-Acetoxy-3-chlor-2,6-dimethyl-octa-1,7-dien oder 3-Chlor-2,6-dimethyl-octa-1,7-dien-6-ol mit einer Base zu 2-Isopropenyl-5-methyl-5-vinyl-tetra-hydrofuran umsetzt.

8. Verfahren nach einem der Ansprüche 1 bis 6, dadurch gekennzeichnet, dass man 6-Acetoxy-3-Chlor-2,6-dimethyl-1-octen mit einer Base zu 2-Aethyl-5-isopropenyl-2-methyl-tetrahydrofuran umsetzt.

9. Verfahren nach einem der Ansprüche 1 bis 6, dadurch gekennzeichnet, dass man 3-Acetoxy-6-chlor-3-methyl-7-methyliden-1-nonen mit einer Base zu 2-Methyl-5-(1-methyliden-propyl)-2-vinyl-tetrahydrofuran umsetzt.

10. Verfahren nach einem der Ansprüche 1 bis 6, dadurch gekennzeichnet, dass man 6-Acetoxy-3-chlor-2-methyl-1-hepten mit einer Base zu 2-Isopropenyl-5-methyl-tetrahydrofuran umsetzt.

11. Verfahren nach einem der Ansprüche 1 bis 6, dadurch gekennzeichnet, dass man 6-Acetoxy-3-chlor-2,6-dimethyl-1-hepten mit einer Base zu 2,2-Dimethyl-5-isopropenyl-tetrahydrofuran umsetzt.

**Claims**

1. A process for the manufacture of compounds of the general formula (I)

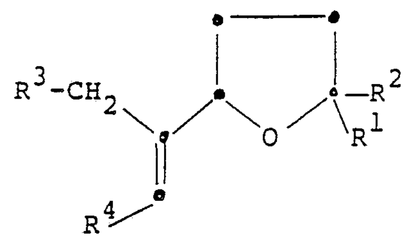

(I)

wherein

$R^1$ signifies hydrogen, $C_{1-6}$-alkyl or $C_{2-6}$-alkenyl,

$R^2$ signifies $C_{1-3}$-alkyl and

$R^3$ and $R^4$ each independently signify hydrogen or $C_{1-3}$-alkyl,

characterized by treating a compound of the general formula (II)

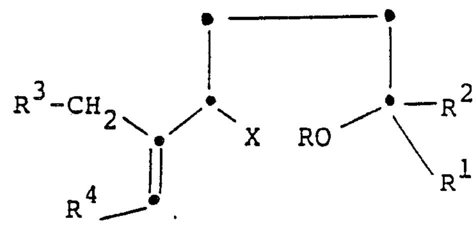

(II)

wherein

$R^1$-$R^4$ have the significances given above,

R signifies hydrogen, formyl, $C_{2-7}$-alkanoyl or aroyl and

X signifies chlorine or bromine,

with a base in the presence of an inert organic solvent.

2. A process according to claim 1, characterized in that an inorganic base is used.

3. A process according to claim 1, characterized in that an organic base is used.

4. A process according to claim 1, 2 or 3, characterized in that the reaction is carried out in an alcohol, an aliphatic ether, a cyclic ether, a saturated aliphatic or alicyclic hydrocarbon or an aromatic hydrocarbon.

5. A process according to any one of claims 1 to 4, characterized in that the reaction is carried out in the presence of a phase transfer catalyst.

6. A process according to any one of claims 1 to 5, characterized in that the reaction is carried out at temperatures between room temperature and 100 °C.

7. A process according to any one of claims 1 to 6, characterized in that 6-acetoxy-3-chloro-2,6-dimethyl-octa-1,7-diene or 3-chloro-2,6-dimethyl-octa-1,7-dien-6-ol is reacted with a base to give 2-isopropenyl-5-methyl-5-vinyl-tetrahydrofuran.

8. A process according to any one of claims 1 to 6, characterized in that 6-acetoxy-3-chloro-2,6-dimethyl-1-octene is reacted with a base to give 2-ethyl-5-isopropenyl-2-methyl-tetrahydrofuran.

9. A process according to any one of claims 1 to 6, characterized in that 3-acetoxy-6-chloro-3-methyl-7-methylidene-1-nonene is reacted with a base to give 2-methyl-5-(1-methylidene-propyl)-2-vinyl-tetrahydrofuran.

10. A process according to any one of claims 1 to 6, characterized in that 6-acetoxy-3-chloro-2-methyl-1-heptene is reacted with a base to give 2-isopropenyl-5-methyl-tetrahydrofuran.

11. A process according to any one of claims 1 to 6, characterized in that 6-acetoxy-3-chloro-2,6-dimethyl-1-heptene is reacted with a base to give 2,2-dimethyl-5-isopropenyl-tetrahydrofuran.

**Revendications**

1. Procédé de préparation de composés de formule générale I

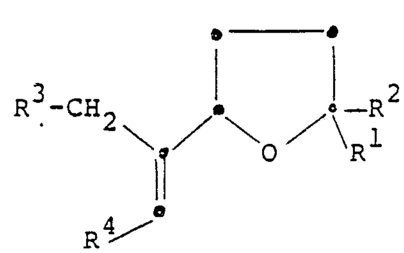

(I)

où

$R^1$ représente un hydrogène, alcoyle en $C_{1-6}$, ou alcényle en $C_{2-6}$,

$R^2$ représente un alcoyle en $C_{1-3}$ et

$R^3$ et $R^4$ représentent indépendamment l'un de l'autre un hydrogène ou un alcoyle en $C_{1-3}$,

caractérisé en ce qu'on traite un composé de formule générale II

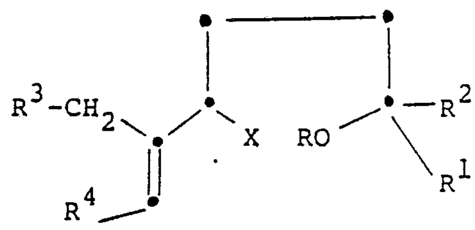

(II)

où

$R^1$-$R^4$ ont les significations données ci-dessus,

R représente un hydrogène, formyle, alcanoyle en $C_2$-$C_7$ ou aroyle, et

X représente un chlore ou un brome

en présence d'un solvant organique inerte, avec une base.

2. Procédé selon la revendication 1, caractérisé en ce que l'on utilise une base inorganique.

3. Procédé selon la revendication 1, caractérisé en ce que l'on utilise une base organique.

4. Procédé selon les revendications 1, 2 ou 3, caractérisé en ce que l'on conduit la réaction dans un alcool, un éther aliphatique, un éther cyclique, un hydrocarbure aliphatique ou alicyclique saturé, ou un hydrocarbure aromatique.

5. Procédé selon l'une des revendications 1 à 4, caractérisé en ce que l'on conduit la réaction en présence d'un catalyseur de transfert de phase.

6. Procédé selon l'une des revendications 1 à 5, caractérisé en ce que l'on conduit la réaction à des températures comprises entre la température ambiante et 100 °C.

7. Procédé selon l'une des revendications 1 à 6, caractérisé en ce que l'on fait réagir le 6-acétoxy-3-chloro-2,6-diméthyl-octa-1,7-diène ou le 3-chloro-2,6-diméthyl-octa-1,7-dién-6-ol avec une base pour donner le 2-isopropényl-5-méthyl-5-vinyl-tétrahydrofuranne.

8. Procédé selon l'une des revendications 1 à 6, caractérisé en ce que l'on fait réagir le 6-acétoxy-3-chloro-2,6-diméthyl-1-octène avec une base pour donner le 2-éthyl-5-isopropényl-2-méthyl-tétrahydrofuranne.

9. Procédé selon l'une des revendications 1 à 6, caractérisé en ce que l'on fait réagir le 3-acétoxy-6-chloro-3-méthyl-7-méthylidène-1-nonène avec une base pour donner le 2-méthyl-5-(1-méthylidène-propyl)-2-vinyl-tétrahydrofuranne.

10. Procédé selon les revendications 1 à 6, caractérisé en ce que l'on fait réagir le 6-acétoxy-3-chloro-2-méthyl-1-heptène avec une base pour donner le 2-isopropényl-5-méthyl-tétrahydrofuranne.

11. Procédé selon l'une des revendications 1 à 6, caractérisé en ce que l'on fait réagir le 6-acétoxy-3-chloro-2,6-diméthyl-1-heptène avec une base pour donner le 2,2-diméthyl-5-isopropényl-tétrahydrofuranne.